# FASCICULE DE BREVET EUROPEEN

(11) **EP 2 644 597 B1**
(45) Date de publication et mention de la délivrance du brevet: **13.01.2016**
(21) Numéro de dépôt: 13161193.1
(22) Date de dépôt: 26.03.2013
(51) Int. Cl.: C07D 223/16, C07C 215/08, C07C 229/34, C07C 255/43

(54) **Nouveau procede de synthese de l'ivabradine et de ses sels d'addition a un acide pharmaceutiquement acceptable**
Neues Syntheseverfahren von Ivabradin und pharmazeutisch verträglichen Säureadditionssalzen davon
Novel method for synthesising ivabradine and its pharmaceutically acceptable acid addition salts

(30) Priorité: 27.03.2012 FR 1252728
(43) Date de publication de la demande: 02.10.2013
(73) Titulaire: Les Laboratoires Servier, 92284 Suresnes Cedex (FR)
(72) Inventeur: Le Flohic, Alexandre, 76640 Fauville en Caux (FR); Grandjean, Mathieu, 76170 La Frenaye (FR)

(56) Documents cités:
- EP-A1- 2 364 972
- WO-A1-2011/033194
- WO-A2-2008/065681
- "IVABRADINE HYDROCHLORIDE ANTIANGINAL HCN (LF CURRENT) BLOCKER", DRUGS OF THE FUTURE, PROUS SCIENCE, ES, vol. 28, no. 7, 1 janvier 2003 (2003-01-01), pages 652-658, XP009052804, ISSN: 0377-8282, DOI: 10.1358/DOF.2003.028.07.742713

## Description

La présente invention concerne un procédé de synthèse de l'ivabradine de formule (I) : 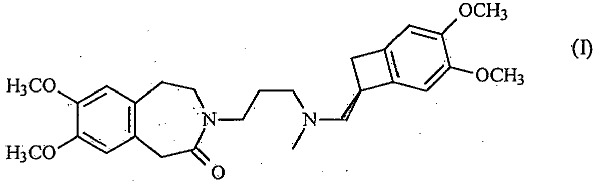 ou 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one,
de ses sels d'addition à un acide pharmaceutiquement acceptable et de leurs hydrates.

L'ivabradine, ainsi que ses sels d'addition à un acide pharmaceutiquement acceptable, et plus particulièrement son chlorhydrate, possèdent des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

La préparation et l'utilisation en thérapeutique de l'ivabradine et de ses sels d'addition à un acide pharmaceutiquement acceptable, et plus particulièrement de son chlorhydrate, ont été décrits dans le brevet européen EP 0 534 859.

Ce brevet décrit la synthèse du chlorhydrate de l'ivabradine à partir du composé de formule (II): 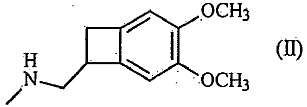 qui est dédoublé pour conduire au composé de formule (III): 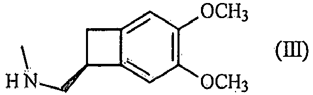 qui est mis en réaction avec le composé de formule (IV) : 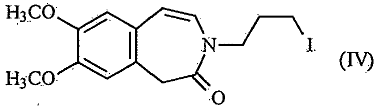 pour conduire au composé de formule (V) : 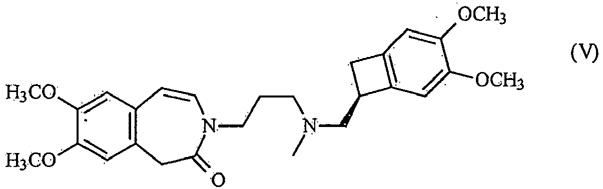 dont l'hydrogénation catalytique conduit à l'ivabradine, qui est alors transformée en son chlorhydrate.

L'inconvénient de cette voie de synthèse est de ne conduire à l'ivabradine qu'avec un rendement de 1%.

La demande WO2008/065 681 décrit une voie de synthèse alternative de l'ivabradine basée sur une réaction d'alkylation entre la 3-chloro-*N*-{[(7*S*)-3,4-diméthoxybicyclo[4.2.0]octa-1,3,5-trién-7-yl]méthyl}-*N*-méthylpropan-1-amine et la 7,8-diméthoxy-1,3,4,5-tétrahydro-2*H-*3-benzazépin-2-one sans qu'aucun rendement ne soit divulgué.

Compte-tenu de l'intérêt pharmaceutique de ce composé, il était important de pouvoir y accéder avec un procédé de synthèse performant, conduisant à l'ivabradine avec un bon rendement.

La présente invention concerne un procédé de synthèse de l'ivabradine de formula (I): 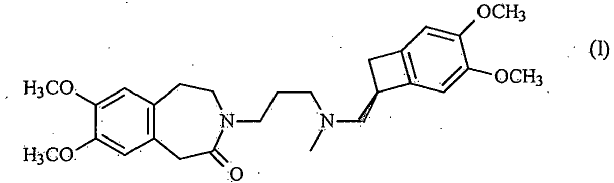 **caractérisé en ce que** le composé de formule (VI): 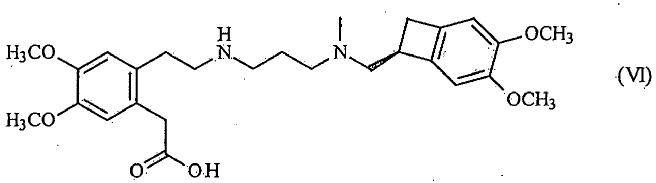 est soumis à une réaction de lactamisation, en présence d'un agent de couplage et d'une base,
dans un solvant organique,
pour conduire à l'ivabradine de formule (I), qui peut être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

Parmi les agents de couplage pouvant être utilisés pour la réaction de lactamisation du composé de formule (VI), on peut citer à titre non limitatif les réactifs suivants : le chlorure d'oxalyle, le chlorure de thionyle, le *N*,*N*-dicyclohexyl carbodiimide (DCC), le 1-éthyl-3-(3-diméthylaminopropyl)carbodiimide (EDCI), le *N*,*N*-carbonyldiimidazole (CDI), l'anhydride cyclique 1-propanephosphonique (T3P) et le 1-(méthylsulfonyl)-1H-benzotriazole.

L'agent de couplage préférentiellement utilisé pour la réaction de lactamisation du composé de formule (VI) est le chlorure de thionyle.

La quantité de chlorure de thionyle préférentiellement utilisée pour effectuer la réaction de lactamisation du composé de formule (VI) est comprise entre 1 et 5 équivalents.

Parmi les bases pouvant être utilisées pour effectuer la réaction de lactamisation du composé de formule (VI), on peut citer à titre non limitatif la triéthylamine, la diisopropyléthylamine et la pyridine.

La base préférentiellement utilisée pour effectuer la réaction de lactamisation du composé de formule (VI) est la triéthylamine.

Parmi les solvants organiques pouvant être utilisés pour effectuer la réaction de lactamisation du composé de formule (VI), on peut citer à titre non limitatif le dichlorométhane, le tétrahydrofurane, l'acétonitrile, l'acétone et le toluène.

Le solvant organique préférentiellement utilisé pour effectuer la réaction de lactamisation du composé de formule (VI) est le dichlorométhane.

La réaction de lactamisation du composé de formule (VI) est préférentiellement conduite à une température comprise entre 0 °C et 40 °C.

La présente invention concerne également un procédé de synthèse de l'ivabradine à partir du composé de formule (VI), **caractérisé en ce que** ledit composé de formule (VI) est préparé à partir du composé de formule (VII) : 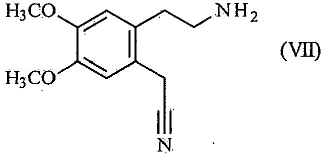 qui est mis en réaction avec le composé de formule (VIII) : 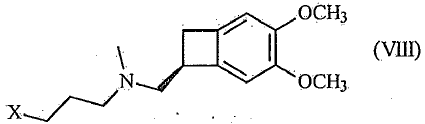 dans laquelle X représente un atome d'halogène, un groupement mésylate ou un groupement tosylate,
en présence d'une base,
dans un solvant organique,
pour conduire au composé de formule (IX): 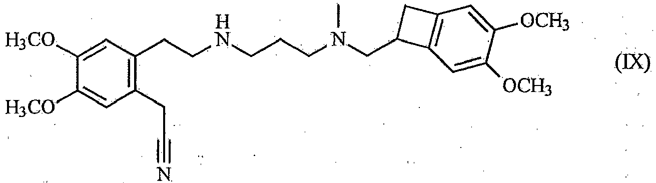 lequel est hydrolysé en composé de formule (VI), par l'action d'une base dans un mélange de solvant organique et d'eau : 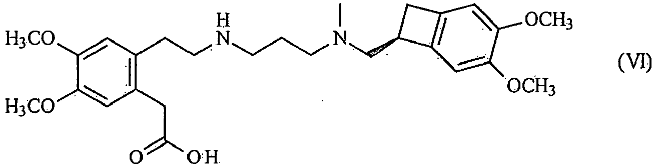 lequel est transformé en ivabradine de formule (I) : 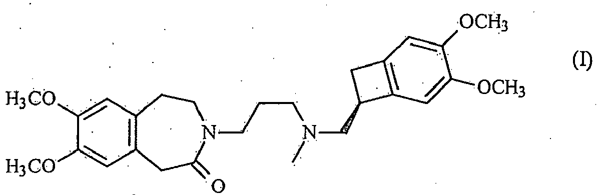 selon le procédé décrit plus haut.

La présente invention concerne également un procédé de synthèse de l'ivabradine à partir du composé de formule (VI), caractérisé **en ce que** ledit composé de formule (VI) est préparé à partir du composé de formule (X) : 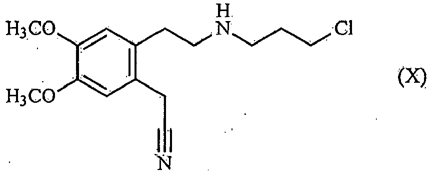 qui est mis en réaction avec le chlorhydrate du composé de formule (III): 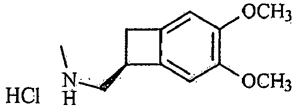 en présence d'une base,
dans un solvant organique,
pour conduire au composé de formule (IX): 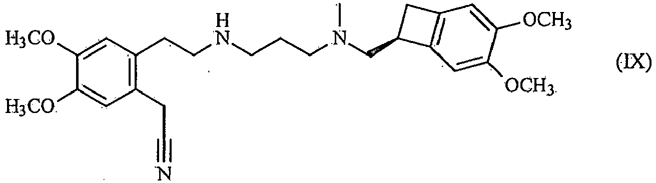 lequel est hydrolysé en composé de formule (VI), par l'action d'une base dans un mélange de solvant organique et d'eau : 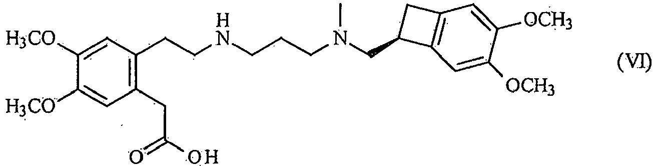 lequel est transformé en ivabradine de formule (I): 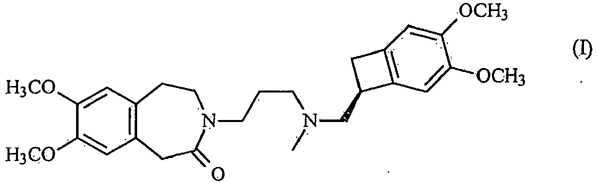 selon le procédé décrit plus haut.

Parmi les bases pouvant être utilisées pour effectuer la réaction d'alkylation entre le composé de formule (VII) et le composé de formule (VIII) ou la réaction d'alkylation entre le composé de formule (X) et le chlorhydrate du composé de formule (III), on peut citer à titre non limitatif des bases inorganiques comme le carbonate de potassium, le carbonate de sodium, le carbonate de césium, l'hydrogénocarbonate de potassium et l'hydrogénocarbonate de sodium, et des bases organiques comme la triéthylamine, la diisopropyléthylamine et la pyridine.

La base préférentiellement utilisée pour effectuer la réaction d'alkylation entre le composé de formule (VII) et le composé de formule (VIII) ou la réaction d'alkylation entre le composé de formule (X) et le chlorhydrate du composé de formule (III) est la triéthylamine.

Parmi les solvants organiques pouvant être utilisés pour effectuer la réaction d'alkylation entre le composé de formule (VII) et le composé de formule (VIII) ou la réaction d'alkylation entre le composé de formule (X) et le chlorhydrate du composé de formule (III), on peut citer à titre non limitatif l'acétonitrile, l'acétone, la méthyléthylcétone (MEK), le dimethylformamide (DMF), la *N*-methylpyrrolidone (NMP) et le diméthylsulfoxyde (DMSO).

Le solvant organique préférentiellement utilisé pour effectuer la réaction d'alkylation entre le composé de formule (VII) par le composé de formule (VIII) ou la réaction d'alkylation entre le composé de formule (X) et le chlorhydrate du composé de formule (III) est l'acétonitrile.

La réaction d'alkylation entre le composé de formule (VII) et le composé de formule (VIII) ou la réaction d'alkylation entre le composé de formule (X) et le chlorhydrate du composé de formule (III) est préférentiellement conduite à une température comprise entre 20°C et 100°C.

Parmi les bases pouvant être utilisées pour effectuer l'hydrolyse du composé de formule (IX) en composé de formule (VI), on peut citer à titre non limitatif l'hydroxyde de potassium, l'hydroxyde de sodium, l'hydroxyde de lithium et l'hydroxyde de barium.

La base préférentiellement utilisée pour effectuer l'hydrolyse du composé de formule (IX) en composé de formule (VI) est l'hydroxyde de sodium.

Le solvant organique préférentiellement utilisé pour effectuer l'hydrolyse du composé de formule (IX) en composé de formule (VI) est un solvant alcoolique.

Parmi les solvants alcooliques pouvant être utilisés pour effectuer l'hydrolyse du composé de formule (IX) en composé de formule (VI), on peut citer à titre non limitatif le méthanol, l'éthanol, l'isopropanol et le butanol.

Le solvant alcoolique préférentiellement utilisé pour effectuer l'hydrolyse du composé de formule (IX) en composé de formule (VI) est l'éthanol.

L'hydrolyse du composé de formule (IX) en composé de formule (VI) est préférentiellement conduite à une température comprise entre 0° C et 110°C.

Les composés de formule (VI), (IX) et (X) ainsi que le 3-[{[(7*S*)-3,4-diméthoxybicyclo[4.2.0]octa-1,3,5-trién-7-yl]méthyl}(méthyl)amino]-1-propanol, l'oxalate du *N*-{2-[2-(cyanométhyl)-4,5-diméthoxyphényl]éthyl}-β-alaninate d'éthyle et le (2-{2-[(3-hydroxypropyl)amino]éthyl}-4,5-diméthoxyphényl)acétonitrile sont des produits nouveaux, utiles comme intermédiaires de synthèse dans l'industrie chimique ou pharmaceutique, notamment dans la synthèse de l'ivabradine, de ses sels d'addition à un acide pharmaceutiquement acceptable et de leurs hydrates, et font à ce titre partie intégrante de la présente invention.

### Liste des abréviations utilisées:

DMF : diméthylformamide
DMSO : diméthylsulfoxyde
RMN : Résonance Magnétique Nucléaire
PF : point de fusion
THF : tétrahydrofurane

Les spectres RMN sont enregistrés sur un appareil Brüker à 400 MHz pour les spectres proton et à 100MHz pour les spectres carbone.
Les déplacements chimiques sont exprimés en ppm (référence interne TMS).
Les abréviations suivantes ont été utilisées pour qualifier les pics : singulet (s), doublet (d), doublet de doublet (dd), triplet (t), quadruplet (q), multiplet (m).

Les exemples ci-dessous illustrent l'invention.

### PREPARATION A :

### N-[2-(3,4-diméthoxyphényl)éthyl]-2,2,2-trifluoroacétamide

A une solution de 2-(3,4-diméthoxyphényl)éthanamine (50 g, 276 mmoles) dans 350 mL d'acétate d'éthyle agitée à température ambiante, est ajoutée, goutte à goutte, une solution d'anhydride trifluoroacétique (46,1 mL, 330 mmoles) dans 40 mL d'acétate d'éthyle. Après 1h de contact à température ambiante, le milieu est hydrolysé par 100 mL d'eau. La phase organique est lavée avec un mélange eau/triéthylamine (100 mL/38,5 mL), 100 mL de solution aqueuse saturée de NaCl puis séchée sur MgSO₄ et mise à sec à sec pour obtenir 65,8 g d'un solide beige correspondant au produit du titre.
Rendement: 86%
PF: 93°C
*RMN ¹H (CDCl₃, 400MHz) :* 2,86 ppm (2H, t) - 3,62 ppm (2H, q) - 3,89 ppm (6H, s) - 6,52 ppm (NH) - 6,71 ppm et 6,84 ppm (3H, m).
*RMN ¹³C (CDCl₃, 100MHz)* : 34,4 ppm (CH₂) - 41,3 ppm (CH₂) - 55,8 ppm (2CH₃) - 111,6 ppm et 111,8 ppm (2CH) - 115,8 ppm (CF₃, q, ¹J(¹⁹F-¹³G)=288Hz) - 120,7 ppm (1CH) - 130,0 ppm, 147,9 ppm et 149,1 ppm (3 Cq) - 157,5 ppm (C=O, ²J(¹⁹F-¹³C)=37Hz).

### PREPARATION B :

### N-{2-[2-(chlorométhyl)-4,5-diméthoxyphényl]éthyl}-2,2,2-trifluoroacétamide

Dans un tricol, mélanger le *N*-[2-(3,4-diméthoxyphényl)éthyl]-2,2,2-trifluoroacétamide (35 g, 126 mmoles), le formaldéhyde aqueux à 37 % (776 mL, 1,014 mole) dans 120 mL de dichlorométhane à 0°C. A ce mélange biphasique ajouter lentement à 0°C, 345 mL de solution aqueuse d'acide chlorhydrique à 37 % et chauffer à 40°C. Après 3h de contact, hydrolyser le milieu par 250 mL d'eau et laver la phase aqueuse par du dichlorométhane (2 x 100 mL). Réunir les phases organiques, les sécher sur MgSO₄ et mettre à sec sous vide pour obtenir une meringue beige (38,2 g). Le produit obtenu est recristallisé dans le toluène pour obtenir 31,5 g d'une poudre blanche correspondant au produit du titre.
Rendement : 77%
PF : 140°C
*RMN ¹H (CDCl₃, 400MHz)* : 2,89 ppm (2H, t) - 3,58 ppm (2H, q) - 3,79 ppm (3H, s) - 3,81 ppm (3H,s) - 4,54 ppm (2H, s) - 6,45 ppm (NH) - 6,60 ppm (1H, s) - 6,77 ppm (1H, s).
*RMN ¹³C (CDCl₃, 100MHz) :* 31,0 ppm (CH₂) - 40,8 ppm (CH₂) - 44,4 ppm (CH₂) - 55,9 ppm (CH₃) - 56,0 ppm (CH₃) - 112,7 ppm (CH) - 113,6 ppm (CH) - 115,8 ppm (CF₃, q, ¹J(¹⁹F-¹³C) =288Hz) - 127,7 ppm, 129,1 ppm, 148,0 ppm et 150,0 ppm (4 Cq) - 157,6 ppm (C=O, ²J(¹⁹F-¹³C)=37Hz).

### PREPARATION C :

### N-{2-[2-(cyanométhyl)-4,5-diméthoxyphényl]éthyl}-2,2,2-trifluoroacétamide

Dans un tricol, agiter à température ambiante une suspension de cyanure de sodium (9,8 g, 200 mmoles) dans 160 mL de DMSO. A la suspension ajouter, goutte à goutte, une solution de *N*-{2-[2-(chlorométhyl)-4,5-diméthoxyphényl]éthyl}-2,2,2-trifluoroacétamide (26 g, 79,8 mmoles) dans 80 mL de DMSO.Après 1h30 de contact à température ambiante, hydrolyser le milieu par 300 mL d'eau et extraire le milieu par du dichlorométhane (3 x 150 mL). Réunir les phases organiques et les laver avec une solution aqueuse à 10 % de NaOAc (150mL), une solution aqueuse saturée de NaCl (4 x 150 mL), puis les sécher sur MgSO₄ et mettre à sec sous vide. Le produit obtenu est recristallisé dans le toluène (66 mL) pour obtenir 12,8 g d'une poudre blanche correspondant au produit du titre.
Rendement : 51%
PF : 131°C
*RMN ¹H (DMSO, 400MHz) :* 2,78 ppm (2H, t) - 3,38 ppm (2H, q) - 3,73 ppm (6H, s) - 3,91 ppm (2H, s) - 6,80 ppm (1H, s) - 6,95 ppm (1H, s) - 9,52 ppm (NH, t).
*RMN ¹³C (DMSO, 100MHz) :* 19,8 ppm (CH₂) - 31,0 ppm (CH₂) - 39.6 ppm (CH₂) - 55.4 ppm (CH₃) - 55.5 ppm (CH₃) - 113.1 ppm (CH) - 113.7 ppm (CH) - 115.9 ppm (CF₃, q, ¹J(¹⁹F-¹³C)=288Hz) - 119.3 ppm, 121.2 ppm, 129.0 ppm, 147.5 ppm et 148.2 ppm (5 Cq) - 156.2 ppm (C=O, ²J(¹⁹F-¹³C)=36Hz).

### PREPARATION D :

### [2-(2-aminoéthyl)-4,5-diméthoxyphényl]acétonitrile

Chauffer à 50°C un mélange de *N*-{2-[2-(cyanométhyl)-4,5-diméthoxyphényl]éthyl}-2,2,2-trifluoroacétamide (20,5 g, 64,8 mmoles), d'éthanol (160 mL), de carbonate de potassium (13,2g, 97,2 mmoles) et d'eau (40 mL). Après 1h30 de contact, extraire le milieu en ajoutant 200 mL de dichlorométhane et 100 mL de solution aqueuse saturée de NaCl. Extraire la phase aqueuse par 100 mL de dichlorométhane. Réunir les phases organiques, les laver avec une solution aqueuse saturée de NaCl (100 mL), les sécher sur MgSO₄ et mettre à sec sous vide pour obtenir 10 g d'une huile jaune correspondant au produit du titre.
Rendement : 70%
PF: 78°C
*RMN ¹H (DMSO, 400MHz) :* 2,61 ppm (2H, m) - 2,72 ppm (2H, m) - 3,40 à 3,00 (NH₂+HDO) - 3,72 ppm (3H, s) -3,73 ppm (3H, s) - 3,90 ppm (2H, s) - 6,81 ppm (1H, s) - 6,92 ppm (1H, s)
*RMN ¹³C (DMSO, 100MHz) :* 20,0 ppm (CH₂) - 35,9 ppm (CH₂) - 42,9 ppm (CH₂) - 55,5 ppm (CH₃) - 55,6 ppm (CH₃) - 113.0 ppm (CH) - 113,7 ppm (CH) - 119,6 ppm, 121,0 ppm, 131,0 ppm, 147,1 ppm et 148,3 ppm (5 Cq).

### PREPARATION E :

### 3-[{[(7S)-3,4-diméthoxybicyclo[4.2.0]octa-1,3,5-trién-7-yl]méthyl}(méthyl)amino]-1-propanol

Chauffer à 50°C un mélange de chlorhydrate de [(7*S*)-3,4-diméthoxybicyclo[4.2.0]octa-1,3,5-trién-7-yl]-*N*-méthylméthanamine (20 g, 82 mmoles), triéthylamine (34,2 mL, 246 mmoles) et 3-bromo-1-propanol (14,8g, 107 mmoles) dans 100 mL THF avec 5 % de DMF. Après 24h de contact, hydrolyser le milieu par 80 mL d'eau et extraire avec 80 mL de dichlorométhane. Laver la phase organique avec une solution aqueuse saturée de NaCl (5 x 60 mL), la sécher sur MgSO₄ et mettre à sec pour obtenir 23,7 g d'une huile jaune correspondant au produit du titre.
Rendement : 96%
*RMN ¹H (CDCl₃, 400MHz) :* 1,66 ppm (2H, m) - 2,31 ppm (3H, s) - 2,50 à 2,70 ppm (5H, m) - 3,21 ppm (1H, dd) - 3,54 ppm (1H, m) - 3,77 ppm (6H, s et 2H, m) - 6,62 ppm (1H, s) - 6,69 ppm (1H, s).
*RMN ¹³C (CDCl₃,100MHz) :* 27,7 ppm (CH₂) - 34,8 ppm (CH₂) - 40,4 ppm (CH) - 42,3 ppm (CH₃) - 56,2 ppm (CH₃) - 56,3 ppm (CH₃) - 59,1 ppm (CH₂) - 62,8 ppm (CH₂) - 64,9 ppm (CH₂) - 106,7 ppm (CH) - 107,4 ppm (CH) - 134,8 ppm, 138,5 ppm, 140,4 ppm, et 149,9 ppm (4 Cq).

### PREPARATION F :

### 3-chloro-N-{[(7S)-3,4-diméthoxybicyclo[4.2.0]octa-1,3,5-trién-7-yl]méthyl}-N-méthyl-1-propanamine

A un mélange de 3-[{[(7*S*)-3,4-diméthoxybicyclo[4.2.0]octa-1,3,5-trién-7-yl]méthyl}(méthyl)amino]-1-propanol (20,8 g, 78,4 mmoles) et de triéthylamine (11 mL, 78,9 mmoles) dans 200 mL de dichlorométhane, ajouter à température ambiante 8,65 mL de chlorure de thionyle (157 mmoles). Après 3h de contact à 40°C, hydrolyser le milieu par 150 mL d'eau et 30 mL de solution aqueuse 1N de NaOH. Ajuster le pH de la phase aqueuse à 10 à l'aide de solution aqueuse 10N de NaOH et l'extraire par 50 mL de dichlorométhane. Réunir les phases organiques et les laver avec une solution aqueuse saturée de Na₂CO₃ (100 mL), les sécher sur MgSO₄ et mettre à sec sous vide pour obtenir 19,8 g d'une huile marron correspondant au produit du titre.
Rendement : 89%
*RMN ¹H (CDCl₃, 400MHz)* : 1,90 ppm (2H, m) - 2,28 ppm (3H, s) - 2,65 ppm (3H, m) - 2,68 ppm (2H, m) - 3,19 ppm (1H, dd) - 3,54 ppm (1H, m) - 3,56 ppm (2H, t) - 3,78 ppm (6H, s) - 6,62 ppm (1H, s) - 6,66 ppm (1H, s).
*RMN ¹³C (CDCl₃, 100MHz) :* 30,2 ppm (CH₂) - 35,0 ppm (CH₂) - 40,6 ppm (CH) - 42,6 ppm (CH₃) - 43,1 ppm (CH₂) - 54,8 ppm (CH₂) - 56,2 ppm (CH₃) - 56,3 (CH₃) - 62,0 ppm (CH₂) - 106,8 ppm (CH) - 107,4 ppm (CH) - 135,0 ppm, 135,0 ppm, 149,3 ppm et 149,9 ppm (4 Cq).

### PREPARATION G :

### Oxalate de 3-chloro-N-{[(7S)-3,4-diméthoxybicyclo[4.2.0]octa-1,3,5-trién-7-yl]méthyl}-N-méthyl-1-propanamine

A une solution de 3-chloro-N-{[(7*S*)-3,4-diméthoxybicyclo[4.2.0]octa-1,3,5-trién-7-yl]méthyl}-*N*-méthyl-1-propanamine (19,8 g, 69,8 mmoles) dans 60 mL d'acétate d'éthyle chauffée au reflux, est ajoutée une solution d'acide oxalique (6,91 g, 76,7 mmoles) dans 60 mL d'éthanol. Le milieu précipite durant le contact au reflux. Après retour à température ambiante, le milieu est filtré et lavé par 20 mL d'éthanol pour obtenir 17,35 g d'une poudre marron correspondant au produit du titre.
Rendement : 67%
PF : 154°C
*RMN ¹H (DMSO, 400MHz) :* 2,14 ppm (2H, m) - 2,75 ppm (3H, s) - 2,88 ppm (1H, dd) - 3,12 ppm (2H, m) - 3,16 ppm (1H, dd) - 3,28 ppm (1H, dd) - 3,41 ppm (1H, dd) - 3,69 à 3,75 ppm (9H, m) - 6,79 ppm (1H, s) - 6,86 ppm (1H, s) - 9,21 ppm (20H).
*RMN ¹³C (DMSO, 100MHz) :* 26,8 ppm (CH₂) - 35,0 ppm (CH₂) - 37,5ppm (CH) - 40,1 ppm (CH₂) - 42,6 ppm (CH₂) - 53,1 ppm (CH₂) - 55,8 ppm (CH₃) - 55,9 ppm (CH₃) - 58,6 ppm (CH₂)- 107,6 ppm (CH) - 108,0 ppm (CH) - 134,2 ppm, 135,7 ppm, 149,3 ppm et 150,2 ppm (4 Cq) - 164,4 ppm (C=O).

### PREPARATION H:

### Oxalate du N-{2-[2-(cyanométhyl)-4,5-diméthoxyphényl]éthyl}-β-alaninate d'éthyle

Un mélange de [2-(2-aminoéthyl)-4,5-diméthoxyphényl]acétonitrile (6,5 g, 29,5 mmol), d'acrylate d'éthyle (3,9 ml, 36 mmol, 1,2éq) dans 120 mL d'éthanol est agité 20h à température ambiante. Le milieu réactionnel est mis à sec sous vide puis le brut réactionnel est repris dans un mélange d'acétate d'éthyle (133 mL) et d'éthanol (13 mL) et chauffé au reflux en présence d'acide oxalique (2,52 g, 28 mmol, 0,95éq). Le milieu précipite durant le contact au reflux. Après retour à température ambiante, le milieu est filtré et lavé par 19 ml d'acétate d'éthyle pour obtenir une poudre blanche (9 g) correspondant au produit du titre.
Rendement : 74%
PF: 218°C
*R:MN ¹H (CDCl₃, 400MHz) :* 1.19 ppm (3H, t) - 2.74 ppm (2H, t) - 2.88 ppm (2H, m) - 3.06 ppm (2H, m) - 3.17 ppm (2H, t) - 3.74 ppm (3H, s) - 3.75 ppm (3H, s) - 3.93 ppm (2H, s) - 4.09 ppm (2H, quadruplet) - 6.88 ppm (1H, s) - 6.97 ppm (1H, s).
*RMN ¹³C (CDCl₃, 100MHz) :* 14.00 ppm (CH₃) - 19.86 ppm (CH₂) - 28.28 ppm (CH₂) - 30.39 ppm (CH₂) - 42.25 ppm (CH₂) - 47.19 ppm (CH₂) - 55.60 ppm (CH₃) - 55.62 ppm (CH₃) - 60.53 ppm (CH₂) - 113.13 ppm (CH) - 113.74 ppm (CH) - 119.36 ppm (Cq)-121.40 ppm (Cq) - 127.70 ppm (Cq) - 147.73 ppm (Cq) - 148.42 ppm (Cq) - 164.65 ppm (Cq) - 170.29 ppm (Cq).

### PREPARATION I :

### (2-{2-[(3-hydroxypropyl)amino]éthyl}-4,5-diméthoxyphényl)acétonitrile

Sur une suspension de 10,8 g de NaBH₄ (284 mmol, 11éq) dans 110 mL de THF, on ajoute en plusieurs fois l'oxalate du *N*-{2-[2-(cyanométhyl)-4,5-diméthoxyphényl]éthyl}-β-alaninate d'éthyle (10,6 g, 25,9 mmol). On laisse agiter 30 min à température ambiante puis 23,1 ml de méthanol (570 mmol, 22éq) sont coulés goutte à goutte. On chauffe le milieu 16h à 60°C puis on l'hydrolyse par 100 mL d'acide chlorhydrique 5N. On ajoute ensuite 100 mL de dichlorométhane et 200 mL d'eau déminéralisée. Après décantation, on ajoute 50 ml de soude 10N (pH > 10) sur la phase aqueuse et on extrait par 3 x 70 mL de dichlorométhane. Les phases organiques sont réunies et lavées par 2 x 75 mL d'une solution aqueuse saturée en NaCl saturé puis séchées sur MgSO₄. Après mise à sec sous vide, on obtient 6,15 g d'une huile incolore correspondant au produit du titre.
Rendement: 85%
*RMN ¹H (CDCl₃, 400MHz) :* 1.54 ppm (2H, quintuplet) - 2.59 ppm (2H, t) - 2.66 ppm (4H, m) - 3.44 ppm (2H, t) - 3.72 ppm (3H, s) - 3.73 ppm (3H, s) - 3.88 ppm (2H, s) - 6.82 ppm (1H, s) - 6.91 ppm (1H, s).
*RMN ¹³C (CDCl₃, 100MHz) :* 19.96 ppm (CH₂) - 32.44 ppm (CH₂) - 32.69 ppm (CH₂) - 46.73 ppm (CH₂) - 50.58 ppm (CH₂) - 55.51 ppm (CH₃) - 55.59 ppm (CH₃) - 59.62 ppm (CH₂) - 112.98 ppm (CH) - 113.65 ppm (CH) - 119.54 ppm (Cq) - 120.92 ppm (Cq) - 131.27 ppm (Cq) - 147.03 ppm (Cq) - 148.23 ppm (Cq).

### PREPARATION J :

### (2-{2-[(3-chloropropyl)amino]éthyl}-4,5-diméthoxyphényl)acétonitrile

Sur un mélange de 2-{2-[(3-hydroxypropyl)amino]éthyl}-4,5-diméthoxyphényl)acétonitrile (1,7g, 6,1 mmol) et de triéthylamine (2,5 ml, 18,3 mmol, 3 éq) dans 16 mL de dichlorométhane, est coulée goutte à goutte une solution constituée de 885 µL de chlorure de thionyle (12,2 mmol, 2 éq) et 1 mL de dichlorométhane. Le milieu est chauffé 2h à 40°C, puis, une fois revenu à température ambiante, hydrolysé par 15 mL d'eau déminéralisée. Après agitation pendant une nuit à température ambiante, 3 mL d'une solution aqueuse de soude 10N (pH > 10) sont ajoutés. Après décantation, la phase organique est prélevée et conservée. La phase aqueuse est extraite par 20 ml de dichloromethane. Les phases organiques sont réunies et lavées par 25 mL d'une solution aqueuse saturée en NaCl, puis séchées sur MgSO₄. Après mise à sec sous vide 1,5g d'une huile marron correspondant au produit du titre sont obtenus.
Rendement: 83%
*RMN ¹H (CDCl₃, 400MHz):* 1.91 ppm (2H, quintuplet) - 2.75 ppm (2H, m) - 2.77 ppm (2H, m) - 2.83 ppm (2H, m) - 3.59 ppm (2H, t) - 3.71 ppm (2H, s) - 3.86 ppm (3H,s) - 3.87 ppm (3H,s) - 6.71 ppm (1H, s) - 5.84 ppm (1H, s).
*RMN ¹³C (CDCl₃, 100MHz)* : 21.11 ppm (CH₂) - 32.73 ppm (CH₂) - 33.16 ppm (CH₂) - 43.04 ppm (CH₂) - 46.88 ppm (CH₂) - 50.45 ppm (CH₂) - 56.03 ppm (CH₃) - 56.08 ppm (CH₃) - 112.17 ppm (CH) -113.08 ppm (CH) - 118.20 ppm (CH) - 120.02 ppm (Cq) - 130.19 ppm (Cq) -147.72 ppm (Cq) - 148.74 ppm (Cq).

### EXEMPLE 1 :

### {2-[2-({3-[{[(7S)-3,4-diméthoxybicyclo[4.2.0]octa-1,3,5-trién-7-yl]méthyl} (méthyl)amino]propyl}amino)éthyl]-4,5-diméthoxyphényl}acétonitrile

### Première variante

Un mélange d'oxalate de 3-chloro-*N*-{[(7*S*)-3,4-diméthoxybicyclo[4.2.0]octa-1,3,5-trién-7-yl]méthyl}-*N*-méthyl-1-propanamine (15 g, 40,1 mmoles) et de 85 mL de solution aqueuse 1N de NaOH dans 150 mL de dichlorométhane est agité 1h à température ambiante. Le milieu est décanté, la phase organique est séchée sur MgSO₄ et mise à sec sous vide pour obtenir 11,3 g d'une huile orange correspondant à la 3-chloro-*N*-{[(7*S*)-3,4-diméthoxybicyclo[4.2.0]octa-1,3,5-trién-7-yl]méthyl}-*N*-méthyl-1-propanamine.

Le produit précédemment obtenu est agité à température ambiante en présence d'iodure de potassium (1,46 g, 8,78 mmoles) dans 200 mL d'acétonitrile. A ce milieu sont successivement ajoutés la triéthylamine (5,6 mL, 40,2 mmoles) puis le [2-(2-aminoéthyl)-4,5-diméthoxyphényl]acétonitrile (8,82 g, 40,1 mmoles) en solution dans 50 mL d'acétonitrile. Après 24h de contact à 60°C, 150 mL d'eau sont ajoutés et le milieu est extrait par ajout de dichlorométhane (150 mL). La phase organique est lavée par 185 mL d'eau et 15 mL de solution aqueuse d'acide chlorhydrique à 37 %. La phase aqueuse est collectée, 185 mL d'une solution aqueuse saturée de NaHCO₃ sont ajoutés, et cette phase est extraite par 150 mL de dichlorométhane. La phase organique est séchée sur MgSO₄ et mise à sec pour obtenir 14, 1 g d'une huile orangée correspondant au produit du titre.
Rendement : 75%

### Deuxième variante

Un mélange de (2-{2-[(3-chloropropyl)amino]éthyl}-4,5-diméthoxyphényl)acétonitrile (870 mg, 2,93 mmol), de chlorhydrate de 1-[(7*S*)-3,4-diméthoxybicyclo[4.2.0]octa-1,3,5-trién-7-yl]-N-méthylméthanamine (714 mg, 2,93 mmol) et de triéthylamine (1,25 ml, 8,97 mmol, 3éq) dans 10 mL d'acétonitrile est chauffé au reflux pendant 6h. Après retour à température ambiante, le milieu est filtré sous vide. Le filtrat est séché sur MgSO₄ et mis à sec sous vide pour obtenir une meringue marron (0.9 g, 66 %) correspondant au produit du titre.
Rendement: 66%
*RMN ¹H (CDCl₃, 400MHz) :* 2,10 ppm (12H, s) - 2,45 ppm (2H, m) - 2,84 à 2,94 ppm (2H, m) - 3,10 à 3,60 ppm (10H, m) 3,80 ppm (6H, m) - 4,36 ppm (NH, s) - 6,56 ppm (1H, s) - 6,69 ppm (1H, s) - 6,78 ppm (1H, s) - 6,83 ppm (1H, s).
*RMN ¹³C (CDCl₃, 100MHz) :* 21,3 ppm (CH₂) - 21,8 ppm CH₂) - 29,0 ppm (CH₂) - 36,2 ppm (CH₂) - 37,5 ppm (CH) - 40,7 ppm (CH₃) - 45,6 ppm (CH₂) - 48,6 ppm (CH₂) - 55,1 ppm (CH₂) - 56,1 ppm (CH₃) - 56,2 ppm (CH₃) - 56,4 ppm (CH₃) - 56,6 ppm (CH₃) - 60,1 ppm (CH₂) - 107,1 ppm (CH) - 107,3 ppm (CH) - 112,7 ppm (CH) - 113,6 ppm (CH) - 119,0 ppm, 120,8 ppm, 126,7 ppm, 134,1 ppm, 134,2 ppm, 148,5 ppm, 149,2 ppm, 149,9 ppm et 150,9 ppm (9C q).

### EXEMPLE 2 :

### Acide {2-[2-({3-[{[(7S)-3,4-diméthoxybicyclo[4.2.0]octa-1,3,5-trién-7-yl]méthyl} (méthyl)amino]propyl}amino)éthyl]-4,5-diméthoxyphényl}acétique

1,3 g de produit obtenu à l'exemple 1 (2,77 mmoles) et 1,11 g de pastilles de NaOH (27.7 mmoles) sont chauffés au reflux dans 6,2 mL d'éthanol et 14,6 mL d'eau. Après 6 h de reflux, le milieu réactionnel est hydrolysé par 10 mL d'eau et 20 mL de dichlorométhane sont ajoutés. La phase organique est extraite par 20 mL d'eau. Les phases aqueuses sont réunies et lavées par 15 mL de dichlorométhane. Le pH de la phase aqueuse lavée est ajusté à 7 à l'aide d'une solution aqueuse d'acide chlorhydrique à 37 % puis mise à sec sous vide. Le solide jaune ainsi obtenu est repris dans 40 mL d'acétone à température ambiante. La suspension obtenue est filtrée sous vide. Les jus de filtration sont mis à sec sous vide pour obtenir 0,7 g d'une meringue jaune correspondant au produit du titre,
Rendement : 52%
*RMN ¹H (CDCl₃, 400MHz) :* 2,10 ppm (2H, m) - 2,54 ppm (3H, s) - 2,70 à 3,30 ppm (12H, m) - 3,47 ppm (2H, s) - 3,55 ppm (1H, m) - 3,71 ppm (3H, s) - 3,73 ppm (3H, s) - 3,74 ppm (3H, s) - 3,75 ppm (3H, s) - 6,55 ppm (H, s) - 6,62 ppm (H, s) - 6,63 ppm (H, s) - 6,64 ppm (H, s).
*RMN ¹³C (CDCl₃, 100MHz) :* 20,7 ppm (CH₂) - 28,1 ppm (CH₂) - 34,9 ppm (CH₂) - 37,0 ppm (CH) - 39,1 ppm (CH₃) - 40,1 ppm (CH₂) - 44,7 ppm (CH₂) - 48,1 ppm (CH₂) - 52,9 ppm (CH₂) - 54,8 ppm (CH₃) - 54,9 ppm (CH₃) - 55,2 ppm (CH₃) - 55,3 ppm (CH₃) - 59,1 ppm (CH₂) - 105,8 ppm (CH) - 106,2 ppm (CH) - 111,6 ppm (CH) - 112,6 ppm (CH) - 126,3 ppm, 127,0 ppm, 133,2 ppm, 134,2 ppm, 146,9 ppm, 147,2 ppm, 148,7 ppm, et 149,6 ppm (8C q) - 176,9 ppm (C=O).

### EXEMPLE 3 :

### 3-{3-[{[(7S)-3,4-diméthoxybicyclo[4.2.0]octa-1,3,5-trién-7-yl]méthyl} (méthyl)amino]propyl}-7,8-diméthoxy-1,3,4,5-tétrahydro-2H-3-benzazépin-2-one

Dans un tricol, mélanger 0,7 g de produit obtenu à l'exemple 2 (1,44 mmoles) et 0,4 mL de triéthylamine (2,88 mmoles) dans 14 mL de dichlorométhane. Glacer le milieu à 5°C et ajouter goutte à goutte 0,16 mL de chlorure de thionyle (2,16 mmoles). Agiter 1h à 30°C puis hydrolyser le milieu par 12 mL de solution aqueuse 1N de NaOH. Laver successivement la phase organique par 10 mL d'eau puis 10 mL de solution aqueuse saturée de NaCl. Sécher la phase organique sur MgSO₄ et mettre à sec sous vide pour obtenir 0,5 g d'une huile orangée correspondant au produit du titre.
Rendement : 74%
*RMN ¹H (CDCl₃, 400MHz) :* 1,78 ppm (2H, m) - 2,32 ppm (3H, s) - 2,40 à 2,80 ppm (5H, m) - 3,16 ppm (2H, t) - 3,19 ppm (1H, m) - 3,42 ppm (3H, m) - 3,55 à 3,80 ppm (16H, m) - 6,50 ppm (1H, s) - 6,52 ppm (1H, s) - 6,61ppm (1H, s) - 6,65 ppm (1H, s).
*RMN ¹³C (CDCl₃, 100MHz) :* 25,0 ppm (CH₂) - 31,3 ppm (CH₂) - 34,3 ppm (CH₂) - 39,2 ppm (CH) - 41,2 ppm (CH₃) - 41,6 ppm (CH₂) - 43,9 ppm (CH₂) - 45,6 ppm (CH₂) - 54,1 ppm (CH₂) - 54,9 ppm (CH₃) - 54,9 ppm (CH₃) - 55,2 ppm (CH₃) - 55,3 ppm (CH₃) - 60,7 ppm (CH₂) - 105,8 ppm (CH) - 106,4 ppm (CH) - 112,1 ppm (CH) - 112,9 ppm (CH) - 122,4 ppm, 126,4 ppm, 126,4 ppm, 133,8 ppm, 146,1 ppm, 146,8 ppm, 148,4 ppm et 148,9 ppm (8C q) -171,2 ppm (C=O).

## Revendications

1. Procédé de synthèse de l'ivabradine de formule (I): 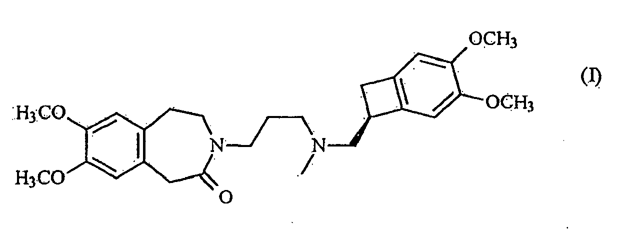 **caractérisé en ce que** le composé de formule (VI): 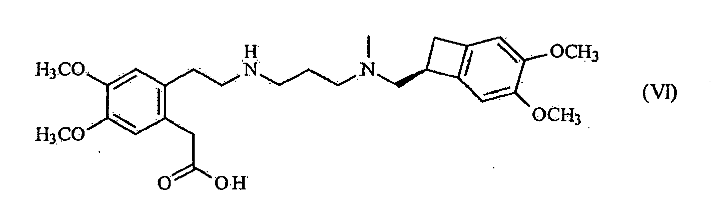 est soumis à une réaction de lactamisation,
en présence d'un agent de couplage et d'une base,
dans un solvant organique,
pour conduire à l'ivabradine de formule (I), qui peut être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

2. Procédé de synthèse selon la revendication 1, **caractérisé en ce que** l'agent de couplage utilisé pour effectuer la réaction de lactamisation du composé de formule (VI) est choisi parmi le chlorure d'oxalyle, le chlorure de thionyle, le *N,N*-dicyclohexyl carbodiimide (DCC), le 1-éthyl-3-(3-diméthylaminopropyl)carbodiimide (EDCI), le *N,N-*carbonyldiimidazole (CDI), l'anhydride cyclique 1-propanephosphonique (T3P) et le 1-(méthylsulfonyl)-1H-benzotriazole.

3. Procédé de synthèse selon la revendication 2, **caractérisé en ce que** l'agent de couplage utilisé pour effectuer la réaction de lactamisation du composé de formule (VI) est le chlorure de thionyle.

4. Procédé de synthèse selon la revendication 3, **caractérisé en ce que** la quantité de chlorure de thionyle utilisée pour effectuer la réaction de lactamisation du composé de formule (VI) est comprise entre 1 et 5 équivalents.

5. Procédé de synthèse selon l'une quelconque des revendications 1 à 4, **caractérisé en ce que** la base utilisée pour effectuer la réaction de lactamisation du composé de formule (VI) est choisie parmi la triéthylamine, la diisopropyléthylamine et la pyridine.

6. Procédé de synthèse selon la revendication 5, **caractérisé en ce que** la base utilisée pour effectuer la réaction de lactamisation du composé de formule (VI) est la triéthylamine.

7. Procédé de synthèse selon l'une quelconque des revendications 1 à 6, **caractérisé en ce que** le solvant organique utilisé pour effectuer la réaction de lactamisation du composé de formule (VI) est choisi parmi le dichlorométhane, le tétrahydrofurane, l'acétonitrile, l'acétone et le toluène.

8. Procédé de synthèse selon la revendication 7, **caractérisé en ce que** le solvant organique utilisé pour effectuer la réaction de lactamisation du composé de formule (VI) est le dichlorométhane.

9. Procédé de synthèse selon l'une quelconque des revendications 1 à 8, **caractérisé en ce que** la réaction de lactamisation du composé de formule (VI) est conduite à une température comprise entre 0°C et 40°C.

10. Procédé de synthèse selon la revendication 1, **caractérisé en ce que** le composé de formule (VI) est préparé à partir du composé de formule (VII): 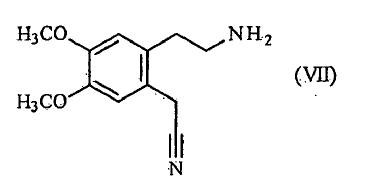 qui est mis en réaction avec le composé de formule (VIII) : 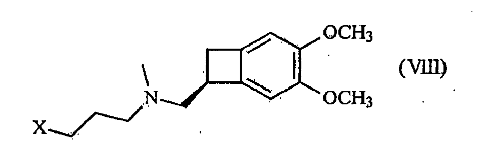 dans laquelle X représente un atome d'halogène, un groupement mésylate ou un groupement tosylate,
en présence d'une base,
dans un solvant organique,
pour conduire au composé de formule (IX): 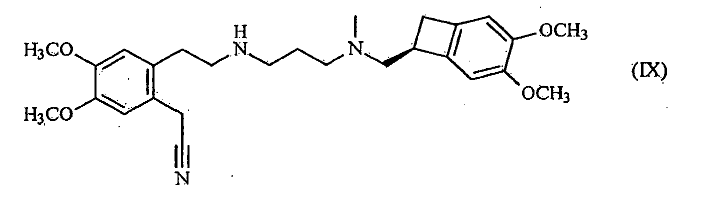 lequel est hydrolysé en composé de formule (VI), par l'action d'une base dans un mélange de solvant organique et d'eau :

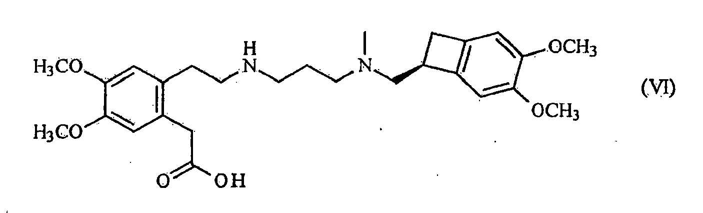

11. Procédé de synthèse selon la revendication 1, **caractérisé en ce que** le composé de formule (VI) est préparé à partir du composé de formule (X) : 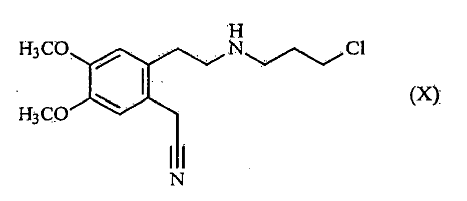 qui est mis en réaction avec le chlorhydrate du composé de formule (III): 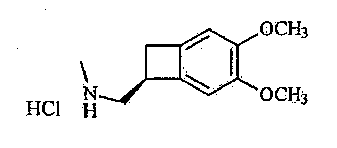 en présence d'une base,
dans un solvant organique,
pour conduire au composé de formule (IX): 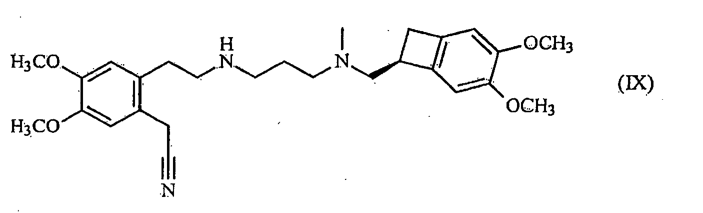 lequel est hydrolysé en composé de formule (VI), par l'action d'une base dans un mélange de solvant organique et d'eau :

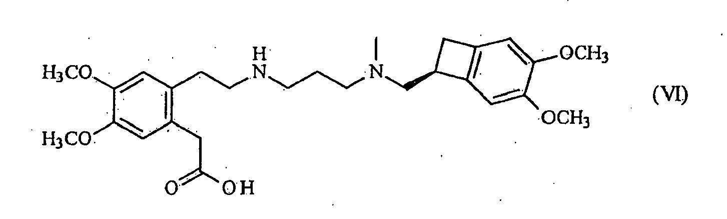

12. Procédé de synthèse selon l'une quelconque des revendications 10 ou 11, **caractérisé en ce que** la base utilisée pour effectuer la réaction d'alkylation entre le composé dé formule et le composé de formule (VIII) ou la réaction d'alkylation entre le composé de formule (X) et le chlorhydrate du composé de formule (III) est choisie parmi le carbonate de potassium, le carbonate de sodium, le carbonate de césium, l'hydrogénocarbonate de potassium, l'hydrogénocarbonate de sodium, la triéthylamine, la diisopropyléthylamine et la pyridine.

13. Procédé de synthèse selon la revendication 12, **caractérisé en ce que** la base utilisée pour effectuer la réaction d'alkylation entre le composé de formule (VII) et le composé de formule (VIII) ou la réaction d'alkylation entre le composé de formule (X) et le chlorhydrate du composé de formule (III) est la triéthylamine.

14. Procédé de synthèse selon l'une quelconque des revendications 10 à 13, **caractérisé en ce que** le solvant organique utilisé pour effectuer la réaction d'alkylation entre le composé de formule (VII) et le composé de formule (VIII) ou la réaction d'alkylation entre le composé de formule (X) et le chlorhydrate du composé de formule (III) est choisi parmi l'acétonitrile, l'acétone, la méthyléthylcétone (MEK), le dimethylformamide (DMF), la *N*-methylpyrrolidone (NMP) et le diméthylsulfoxyde (DMSO).

15. Procédé de synthèse selon la revendication 14, **caractérisé en ce que** le solvant organique utilisé pour effectuer la réaction d'alkylation entre le composé de formule (VII) et le composé de formule (VIII) ou la réaction d'alkylation entre le composé de formule (X) et le chlorhydrate du composé de formule (III) est l'acétonitrile.

16. Procédé de synthèse selon l'une quelconque des revendications 10 à 15, **caractérisé en ce que** la réaction d'alkylation entre le composé de formule (VII) et le composé de formule (VIII) ou la réaction d'alkylation entre le composé de formule (X) et le chlorhydrate du composé de formule (III) est conduite à une température comprise entre 20°C et 100 °C.

17. Procédé de synthèse selon l'une quelconque des revendications 10 à 16, **caractérisé en ce que** la base utilisée pour effectuer l'hydrolyse du composé de formule (IX) en composé de formule (VI) est choisie parmi l'hydroxyde de potassium, l'hydroxyde de sodium, l'hydroxyde de lithium et l'hydroxyde de barium.

18. Procédé de synthèse selon la revendication 17, **caractérisé en ce que** la base utilisée pour effectuer l'hydrolyse du composé de formule (IX) en composé de formule (VI) est l'hydroxyde de sodium.

19. Procédé de synthèse selon l'une quelconque des revendications 10 à 18, **caractérisé en ce que** le solvant organique utilisé pour effectuer l'hydrolyse du composé de formule (IX) en composé de formule (VI) est un solvant alcoolique.

20. Procédé de synthèse selon la revendication 19, **caractérisé en ce que** le solvant alcoolique utilisé pour effectuer l'hydrolyse du composé de formule (IX) en composé de formule (VI) est choisi parmi le méthanol, l'éthanol, l'isopropanol et le butanol.

21. Procédé de synthèse selon la revendication 20, **caractérisé en ce que** le solvant alcoolique utilisé pour effectuer l'hydrolyse du composé de formule (IX) en composé de formule (VI) est l'éthanol.

22. Procédé de synthèse selon l'une quelconque des revendications 10 à 21, **caractérisé en ce que** l'hydrolyse du composé de formule (IX) en composé de formule (VI) est conduite à une température comprise entre 0°C et 110°C.

23. Composé de formule (VI) :

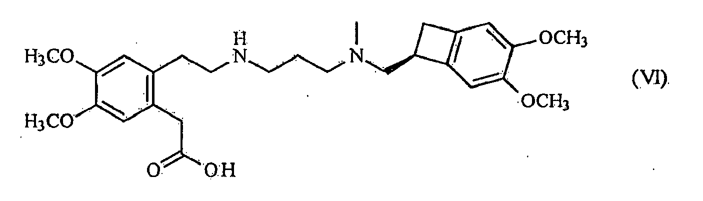

24. Composé de formule (IX) :

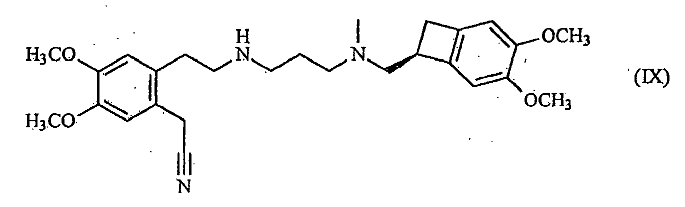

25. Composé de formule (X) :

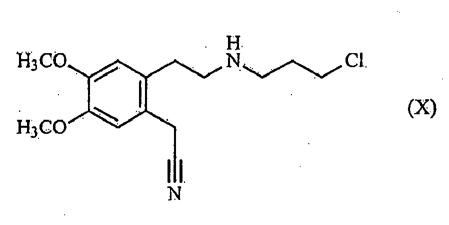

26. Oxalate du *N*-{2-[2-(cyanométhyl)-4,5-diméthoxyphényl]éthyl}-β-alaninate d'éthyle

27. (2-{2-[(3-hydroxypropyl)amino]éthyl}-4,5-diméthoxyphényl)acétonitrile

## Patentansprüche

1. Verfahren zur Synthese von Ivabradin der Formel (I): 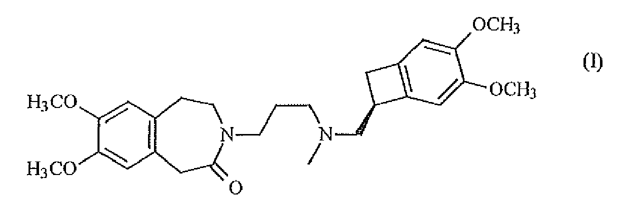 **dadurch gekennzeichnet, dass** die Verbindung der Formel (VI): 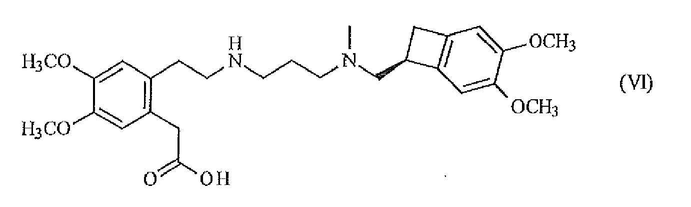 einer Lactamisierungsreaktion,
in Gegenwart eines Kupplungsmittels und einer Base,
in einem organischen Lösungsmittel unterworfen wird
zur Bildung von Ivabradin der Formel (I), welches in seine Additionssalze mit einer pharmazeutisch annehmbaren Säure, ausgewählt aus Chlorwasserstoffsäure, Bromwasserstoffsäure, Schwefelsäure, Phosphorsäure, Essigsäure, Trifluoressigsäure, Milchsäure, Brenztraubensäure, Malonsäure, Bernsteinsäure, Glutarsäure, Fumarsäure, Weinsäure, Maleinsäure, Citronensäure, Ascorbinsäure, Oxalsäure, Methansulfonsäure, Benzolsulfonsäure und Camphersäure, sowie in deren Hydrate umgewandelt werden kann.

2. Syntheseverfahren nach Anspruch 1, **dadurch gekennzeichnet, dass** das zur Durchführung der Reaktion der Lactamisierung der Verbindung der Formel (VI) verwendete Kupplungsmittel ausgewählt wird aus Oxalylchlorid, Thionylchlorid, *N,N-*Dicyclohexylcarbodiimid (DCC), 1-Ethyl-3-(3-dimethylaminopropyl)-carbodiimid (EDCI), *N,N*-Carbonyldiimidazol (CDI), cyclischem 1-Propanphosphonsäureanhydrid (T3P) und 1-(Methylsulfonyl)-1H-benzotriazol.

3. Syntheseverfahren nach Anspruch 2, **dadurch gekennzeichnet, dass** das zur Durchführung der Reaktion der Lactamisierung der Verbindung der Formel (VI) verwendete Kupplungsmittel Thionylchlorid ist.

4. Syntheseverfahren nach Anspruch 3, **dadurch gekennzeichnet, dass** die Menge des zur Durchführung der Reaktion der Lactamisierung der Verbindung der Formel (VI) verwendeten Thionylchlorids zwischen 1 und 5 Äquivalenten liegt.

5. Syntheseverfahren nach einem der Ansprüche 1 bis 4, **dadurch gekennzeichnet, dass** die zur Durchführung der Reaktion der Lactamisierung der Verbindung der Formel (VI) verwendete Base aus Triethylamin, Diisopropylethylamin und Pyridin ausgewählt wird.

6. Syntheseverfahren nach Anspruch 5, **dadurch gekennzeichnet, dass** die zur Durchführung der Reaktion der Lactamisierung der Verbindung der Formel (VI) verwendete Base Triethylamin ist.

7. Syntheseverfahren nach einem der Ansprüche 1 bis 6, **dadurch gekennzeichnet, dass** das zur Durchführung der Reaktion der Lactamisierung der Verbindung der Formel (VI) verwendete organische Lösungsmittel aus Dichlormethan, Tetrahydrofuran, Acetonitril, Aceton und Toluol ausgewählt wird.

8. Syntheseverfahren nach Anspruch 7, **dadurch gekennzeichnet, dass** das zur Durchführung der Reaktion der Lactamisierung der Verbindung der Formel (VI) verwendete organische Lösungsmittel Dichlormethan ist.

9. Syntheseverfahren nach einem der Ansprüche 1 bis 8, **dadurch gekennzeichnet, dass** die Reaktion der Lactamisierung der Verbindung der Formel (VI) bei einer Temperatur zwischen 0 °C und 40 °C durchgeführt wird.

10. Syntheseverfahren nach Anspruch 1, **dadurch gekennzeichnet, dass** die Verbindung der Formel (VI) ausgehend von der Verbindung der Formel (VII) hergestellt wird: 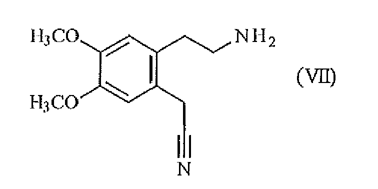 welche mit der Verbindung der Formel (VIII): 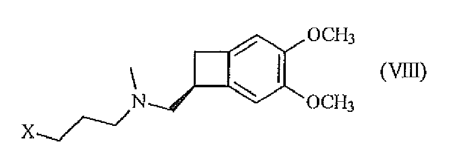 in der X ein Halogenatom, eine Mesylat- oder eine Tosylatgruppe bedeutet,
in Gegenwart einer Base,
in einem organischen Lösungsmittel umgesetzt wird zur Bildung der Verbindung der Formel (IX): 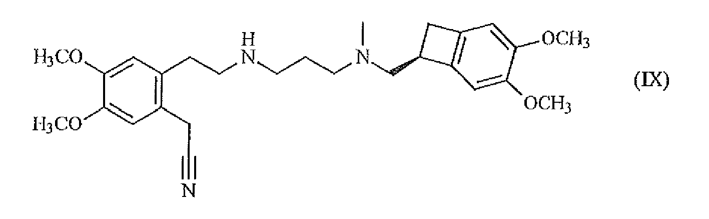 welche durch Einwirkung einer Base in einer Mischung aus organischem Lösungsmittel und Wasser zu der Verbindung der Formel (VI) hydrolysiert wird:

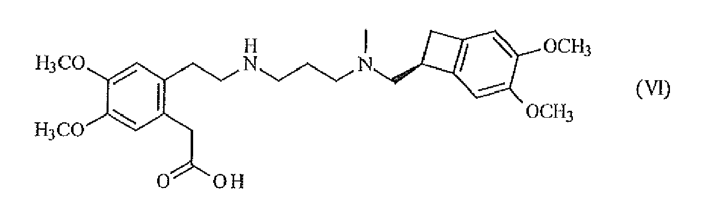

11. Syntheseverfahren nach Anspruch 1, **dadurch gekennzeichnet, dass** die Verbindung der Formel (VI) ausgehend von der Verbindung der Formel (X): 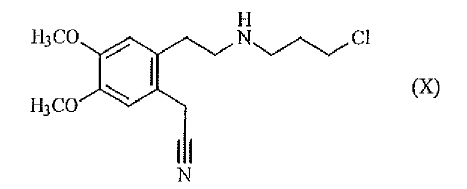 hergestellt wird, welche mit dem Hydrochlorid der Verbindung der Formel (III): 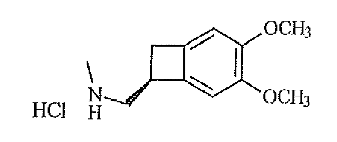 in Gegenwart einer Base
in einem organischen Lösungsmittel umgesetzt wird,
zur Bildung der Verbindung der Formel (IX): 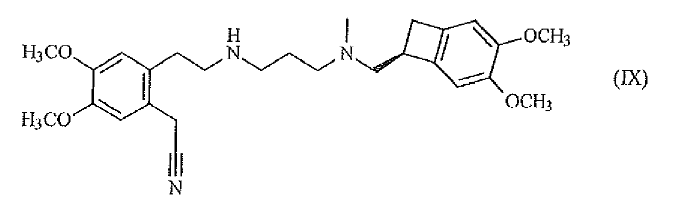 welche durch Einwirkung einer Base in einer Mischung aus organischem Lösungsmittel und Wasser zu der Verbindung der Formel (VI) hydrolysiert wird:

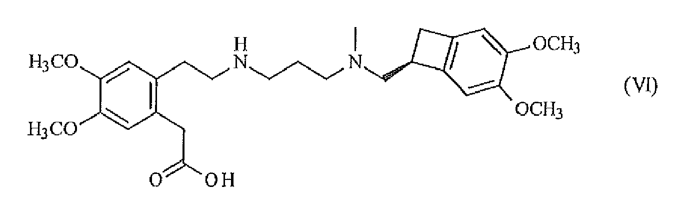

12. Syntheseverfahren nach einem der Ansprüche 10 oder 11, **dadurch gekennzeichnet, dass** die zur Durchführung der Alkylierungsreaktion zwischen der Verbindung der Formel (VII) und der Verbindung der Formel (VIII) oder der Alkylierungsreaktion zwischen der Verbindung der Formel (X) und dem Hydrochlorid der Verbindung der Formel (III) verwendete Base aus Kaliumcarbonat, Natriumcarbonat, Cäsiumcarbonat, Kaliumhydrogencarbonat, Natriumhydrogencarbonat, Triethylamin, Diisopropylethylamin und Pyridin ausgewählt wird.

13. Syntheseverfahren nach Anspruch 12, **dadurch gekennzeichnet, dass** die zur Durchführung der Alkylierungsreaktion zwischen der Verbindung der Formel (VII) und der Verbindung der Formel (VIII) oder der Alkylierungsreaktion zwischen der Verbindung der Formel (X) und dem Hydrochlorid der Verbindung der Formel (III) verwendete Base Triethylamin ist.

14. Syntheseverfahren nach einem der Ansprüche 10 bis 13, **dadurch gekennzeichnet, dass** das zur Durchführung der Alkylierungsreaktion zwischen der Verbindung der Formel (VII) und der Verbindung der Formel (VIII) oder der Alkylierungsreaktion zwischen der Verbindung der Formel (X) und dem Hydrochlorid der Verbindung der Formel (III) verwendete organische Lösungsmittel ausgewählt wird aus Acetonitril, Aceton, Methylethylketon (MEK), Dimethylformamid (DMF), *N*-Methylpyrrolidon (NMP) und Dimethylsulfoxid (DMSO).

15. Syntheseverfahren nach Anspruch 14, **dadurch gekennzeichnet, dass** das zur Durchführung der Alkylierungsreaktion zwischen der Verbindung der Formel (VII) und der Verbindung der Formel (VIII) oder der Alkylierungsreaktion zwischen der Verbindung der Formel (X) und dem Hydrochlorid der Verbindung der Formel (III) verwendete organische Lösungsmittel Acetonitril ist.

16. Syntheseverfahren nach einem der Ansprüche 10 bis 15, **dadurch gekennzeichnet, dass** die Alkylierungsreaktion zwischen der Verbindung der Formel (VII) und der Verbindung der Formel (VIII) oder die Alkylierungsreaktion zwischen der Verbindung der Formel X) und dem Hydrochlorid der Verbindung der Formel (III) bei einer Temperatur zwischen 20 °C und 100 °C durchgeführt wird.

17. Syntheseverfahren nach einem der Ansprüche 10 bis 16, **dadurch gekennzeichnet, dass** die zur Durchführung der Hydrolyse der Verbindung der Formel (IX) zu der Verbindung der Formel (VI) verwendete Base aus Kaliumhydroxid, Natriumhydroxid, Lithiumhydroxid und Bariumhydroxid ausgewählt wird.

18. Syntheseverfahren nach Anspruch 17, **dadurch gekennzeichnet, dass** die zur Durchführung der Hydrolyse der Verbindung der Formel (IX) zu der Verbindung der Formel (VI) verwendete Base Natriumhydroxid ist.

19. Syntheseverfahren nach einem der Ansprüche 10 bis 18, **dadurch gekennzeichnet, dass** das zur Durchführung der Hydrolyse der Verbindung der Formel (IX) zu der Verbindung der Formel (VI) verwendete organische Lösungsmittel ein alkoholisches Lösungsmittel ist.

20. Syntheseverfahren nach Anspruch 19, **dadurch gekennzeichnet, dass** das zur Durchführung der Hydrolyse der Verbindung der Formel (IX) zu der Verbindung der Formel (VI) verwendete alkoholische Lösungsmittel aus Methanol, Ethanol, Isopropanol und Butanol ausgewählt wird.

21. Syntheseverfahren nach Anspruch 20, **dadurch gekennzeichnet, dass** das zur Durchführung der Hydrolyse der Verbindung der Formel (IX) zu der Verbindung der Formel (VI) verwendete alkoholische Lösungsmittel Ethanol ist.

22. Syntheseverfahren nach einem der Ansprüche 10 bis 21, **dadurch gekennzeichnet, dass** die Hydrolyse der Verbindung der Formel (IX) zu der Verbindung der Formel (VI) bei einer Temperatur zwischen 0 °C und 110 °C durchgeführt wird.

23. Verbindung der Formel (VI):

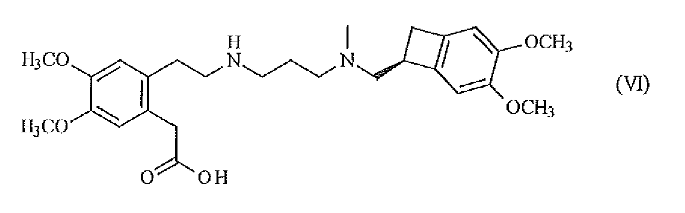

24. Verbindung der Formel (IX):

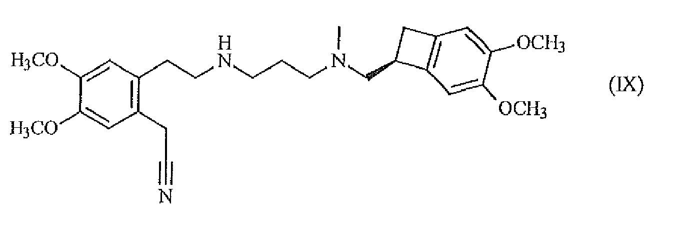

25. Verbindung der Formel (X):

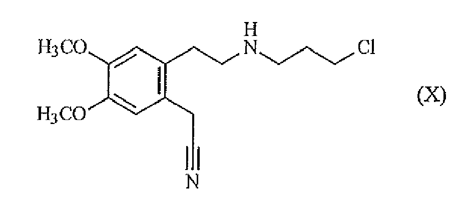

26. Oxalat von *N*-{2-[2-(Cyanomethyl)-4,5-dimethoxyphenyl]-ethyl}-β-alaninsäureethylester.

27. (2-{2-[(3-Hydroxypropyl)-amino]-ethyl}-4,5-dimethoxyphenyl)-acetonitril.

## Claims

1. Process for the synthesis of ivabradine of formula (I): 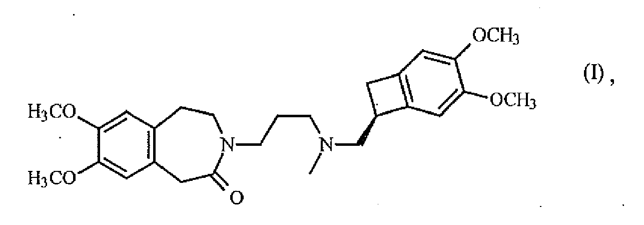 **characterised in that** the compound of formula (VI): 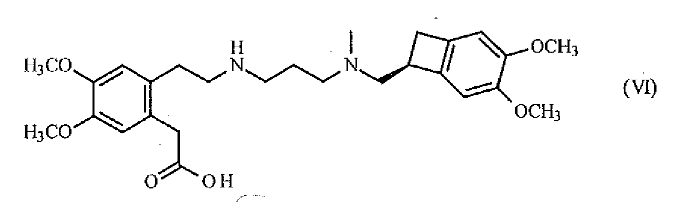 is subjected to a lactamisation reaction,
in the presence of a coupling agent and a base,
in an organic solvent,
to yield ivabradine of formula (I), which may be converted into addition salts thereof with a pharmaceutically acceptable acid selected from hydrochloric acid, hydrobromic acid, sulphuric acid, phosphoric acid, acetic acid, trifluoroacetic acid, lactic acid, pyruvic acid, malonic acid, succinic acid, glutaric acid, fumaric acid, tartaric acid, maleic acid, citric acid, ascorbic acid, oxalic acid, methanesulphonic acid, benzenesulphonic acid and camphoric acid, and into hydrates thereof.

2. Synthesis process according to claim 1, **characterised in that** the coupling agent used to carry out the reaction for lactamisation of the compound of formula (VI) is selected from oxalyl chloride, thionyl chloride, *N*,*N*-dicyclohexylcarbodiimide (DCC), 1-ethyl-3-(3-dimethylaminopropyl)carbodiimide (EDCI), *N*,*N*-carbonyldiimidazole (CDI), 1-propanephosphonic acid cyclic anhydride (T3P) and 1-(methylsulphonyl)-1H-benzotriazole.

3. Synthesis process according to claim 2, **characterised in that** the coupling agent used to carry out the reaction for lactamisation of the compound of formula (VI) is thionyl chloride.

4. Synthesis process according to claim 3, **characterised in that** the amount of thionyl chloride used to carry out the reaction for lactamisation of the compound of formula (VI) is between 1 and 5 equivalents, inclusive.

5. Synthesis process according to any one of claims 1 to 4, **characterised in that** the base used to carry out the reaction for lactamisation of the compound of formula (VI) is selected from triethylamine, diisopropylethylamine and pyridine.

6. Synthesis process according to claim 5, **characterised in that** the base used to carry out the reaction for lactamisation of the compound of formula (VI) is triethylamine.

7. Synthesis process according to any one of claims 1 to 6, **characterised in that** the organic solvent used to carry out the reaction for lactamisation of the compound of formula (VI) is selected from dichloromethane, tetrahydrofuran, acetonitrile, acetone and toluene.

8. Synthesis process according to claim 7, **characterised in that** the organic solvent used to carry out the reaction for lactamisation of the compound of formula (VI) is dichloromethane.

9. Synthesis process according to any one of claims 1 to 8, **characterised in that** the reaction for lactamisation of the compound of formula (VI) is carried out at a temperature between 0°C and 40°C, inclusive.

10. Synthesis process according to claim 1, **characterised in that** the compound of formula (VI) is prepared starting from the compound of formula (VII): 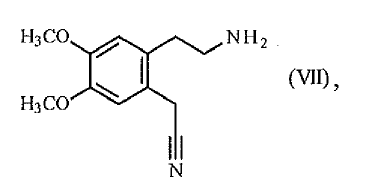 which is reacted with the compound of formula (VIII): 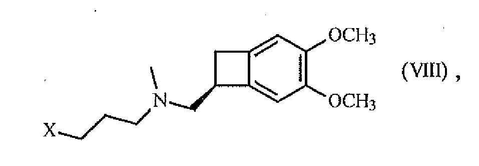 wherein X represents a halogen atom, a mesylate group or a tosylate group,
in the presence of a base,
in an organic solvent,
to yield the compound of formula (IX): 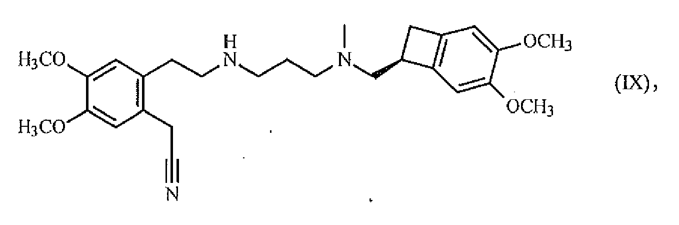 which is hydrolysed, by the action of a base in a mixture of organic solvent and water, to form the compound of formula (VI):

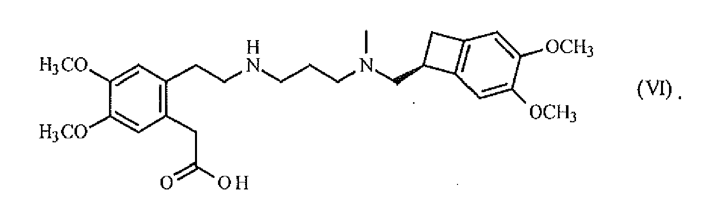

11. Synthesis process according to claim 1, **characterised in that** the compound of formula (VI) is prepared starting from the compound of formula (X): 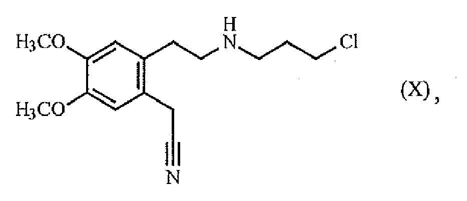 which is reacted with the hydrochloride of the compound of formula (III): 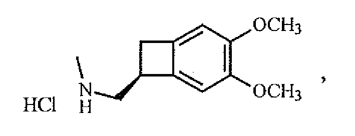 in the presence of a base,
in an organic solvent,
to yield the compound of formula (IX): 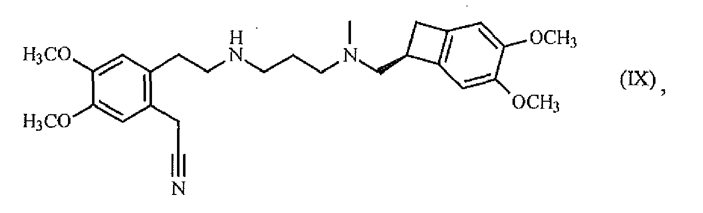 which is hydrolysed, by the action of a base in a mixture of organic solvent and water, to form the compound of formula (VI):

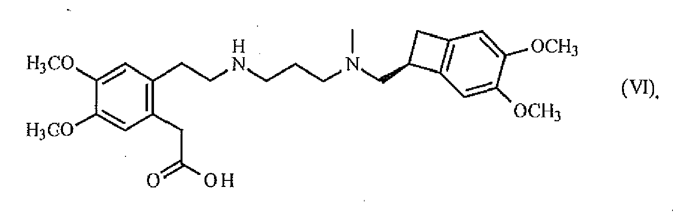

12. Synthesis process according to either claim 10 or claim 11, **characterised in that** the base used to carry out the alkylation reaction between the compound of formula (VII) and the compound of formula (VIII) or the alkylation reaction between the compound of formula (X) and the hydrochloride of the compound of formula (III) is selected from potassium carbonate, sodium carbonate, caesium carbonate, potassium hydrogen carbonate, sodium hydrogen carbonate, triethylamine, diisopropylethylamine and pyridine.

13. Synthesis process according to claim 12, **characterised in that** the base used to carry out the alkylation reaction between the compound of formula (VII) and the compound of formula (VIII) or the alkylation reaction between the compound of formula (X) and the hydrochloride of the compound of formula (III) is triethylamine.

14. Synthesis process according to any one of claims 10 to 13, **characterised in that** the organic solvent used to carry out the alkylation reaction between the compound of formula (VII) and the compound of formula (VIII) or the alkylation reaction between the compound of formula (X) and the hydrochloride of the compound of formula (III) is selected from acetonitrile, acetone, methyl ethyl ketone (MEK), dimethylformamide (DMF), N-methylpyrrolidone (NMP) and dimethyl sulphoxide (DMSO).

15. Synthesis process according to claim 14, **characterised in that** the organic solvent used to carry out the alkylation reaction between the compound of formula (VII) and the compound of formula (VIII) or the alkylation reaction between the compound of formula (X) and the hydrochloride of the compound of formula (III) is acetonitrile.

16. Synthesis process according to any one of claims 10 to 15, **characterised in that** the alkylation reaction between the compound of formula (VII) and the compound of formula (VIII) or the alkylation reaction between the compound of formula (X) and the hydrochloride of the compound of formula (III) is carried out at a temperature between 20°C and 100°C, inclusive.

17. Synthesis process according to any one of claims 10 to 16, **characterised in that** the base used to carry out the hydrolysis of the compound of formula (IX) to form the compound of formula (VI) is selected from potassium hydroxide, sodium hydroxide, lithium hydroxide and barium hydroxide.

18. Synthesis process according to claim 17, **characterised in that** the base used to carry out the hydrolysis of the compound of formula (IX) to form the compound of formula (VI) is sodium hydroxide.

19. Synthesis process according to any one of claims 10 to 18, **characterised in that** the organic solvent used to carry out the hydrolysis of the compound of formula (IX) to form the compound of formula (VI) is an alcoholic solvent.

20. Synthesis process according to claim 19, **characterised in that** the alcoholic solvent used to carry out the hydrolysis of the compound of formula (IX) to form the compound of formula (VI) is selected from methanol, ethanol, isopropanol and butanol.

21. Synthesis process according to claim 20, **characterised in that** the alcoholic solvent used to carry out the hydrolysis of the compound of formula (IX) to form the compound of formula (VI) is ethanol.

22. Synthesis process according to any one of claims 10 to 21, **characterised in that** the hydrolysis of the compound of formula (IX) to form the compound of formula (VI) is carried out at a temperature between 0°C and 110°C, inclusive.

23. Compound of formula (VI):

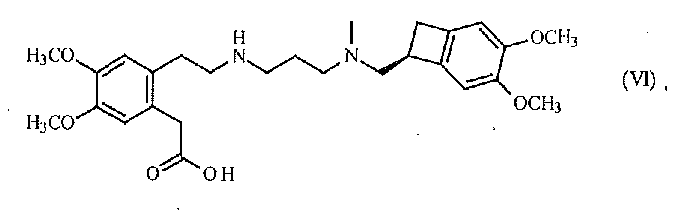

24. Compound of formula (IX):

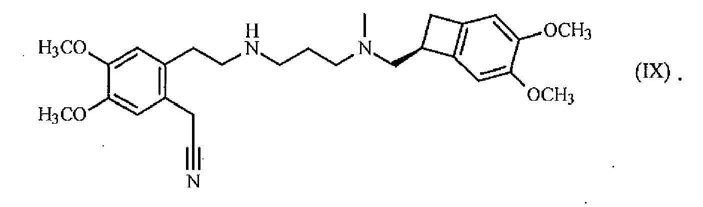

25. Compound of formula (X):

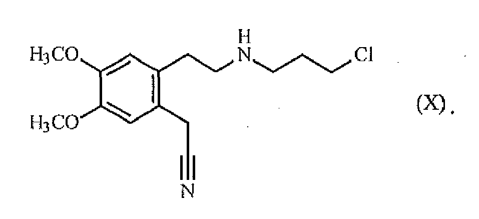

26. Ethyl *N*-{2-[2-(cyanomethyl)-4,5-dimethoxyphenyl]ethyl}-β-alaninate oxalate.

27. (2-{2-[(3-hydroxypropyl)amino]ethyl}-4,5-dimethoxyphenyl)acetonitrile.
